# EUROPÄISCHE PATENTSCHRIFT

(11) **EP 1 139 992 B1**
(45) Veröffentlichungstag und Bekanntmachung des Hinweises auf die Patenterteilung: **19.11.2003**
(21) Anmeldenummer: 99967935.0
(22) Anmeldetag: 14.12.1999
(51) Int. Cl.: A61K 7/13

(54) **FÄRBEMITTEL MIT IN HOHLRÄUMEN VON KÄFIGVERBINDUNGEN (Z.B. ZEOLITHE) EINGESCHLOSSENEN ÜBERGANGSMETALL-KOMPLEXEN**
COLORING AGENT COMPRISING TRANSITION METALS
COLORANTS CONTENANT DES METAUX DE TRANSITION

(30) Priorität: 23.12.1998 DE 19859681
(43) Veröffentlichungstag der Anmeldung: 10.10.2001
(73) Patentinhaber: Henkel Kommanditgesellschaft auf Aktien, 40589 Düsseldorf-Holthausen (DE)
(72) Erfinder: MAYER, Bernd, D-40593 Düsseldorf (DE); KUHM, Peter, D-40724 Hilden (DE); HÖFFKES, Horst, D-40595 Düsseldorf (DE); EWALD, Gertrud, D-42655 Solingen (DE); STEPHAN, Hans-Oscar, D-47269 Duisburg (DE); HELLER, Melita, D-40591 Düsseldorf (DE)
(86) Internationale Anmeldenummer: EP9909903
(87) Internationale Veröffentlichungsnummer: WO00038630

(56) Entgegenhaltungen:
- EP-A- 0 507 448
- EP-A- 0 621 029
- DE-A- 3 642 097
- DE-A- 19 756 455
- DE-A- 19 757 510
- DE-C- 19 534 214
- FR-A- 1 439 307
- US-A- 3 429 646
- US-A- 5 715 845
- DATABASE WPI Section Ch, Week 199006 Derwent Publications Ltd., London, GB; Class D22, AN 1990-040675 XP002134574 & JP 01 317443 A (NIPPON ZEON KK), 22. Dezember 1989 (1989-12-22)
- DATABASE WPI Section Ch, Week 199150 Derwent Publications Ltd., London, GB; Class D21, AN 1991-365788 XP002134575 & JP 03 246217 A (NOEVIER KK), 1. November 1991 (1991-11-01)
- PATENT ABSTRACTS OF JAPAN vol. 010, no. 235 (C-366), 14. August 1986 (1986-08-14) & JP 61 069718 A (HIDETOSHI TSUCHIDA), 10. April 1986 (1986-04-10)

## Beschreibung

Die vorliegende Erfindung betrifft Färbemittel, die in Käfigverbindungen eingeschlossene Übergangsmetallkomplexe als Oxidationskatalysatoren enthalten sowie die Verwendung dieser Mittel zum Färben keratinhaltiger Fasern.

Für das Färben von keratinhaltigen Fasern, z.B. Wolle, Pelzen und insbesondere menschlichen Haaren, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung.

Direktziehende Farbstoffe werden unter schonenden Bedingungen appliziert. Ihr Nachteil liegt jedoch darin, daß die Färbungen häufig nur über unzureichende Echtheitseigenschaften verfügen. Mit Oxidationsfarbstoffen lassen sich zwar intensive Färbungen mit guten Echtheitseigenschaften erzielen, die Entwicklung der Farbe geschieht jedoch in der Regel unter dem Einfluß von H₂O₂ oder H₂O₂-Addukten, was Schädigungen der Faser zur Folge haben kann.

Auch Oxidationsfärbemittel mit Luftsauerstoff als sehr mildem Oxidationsmittel wurden bereits vorgeschlagen; im allgemeinen laufen die Oxidationen mit Luftsauerstoff jedoch nicht vollständig ab. Insbesondere in Oxidationshaarfärbemitteln, die in der Regel in einem cremeartigen kosmetischen Träger appliziert werden, stößt der Luftsauerstoff auf erhebliche Diffusionsbarrieren. Weiterhin wurde, beispielsweise in der EP-B1-0 548 620, vorgeschlagen, den Gehalt an Wasserstoffperoxid in den Oxidationsfärbemitteln auf 1 Gew.-% und weniger zu begrenzen, dem Mittel aber gleichzeitig zur Aktivierung spezielle Enzyme, Peroxidasen, zuzufügen. Alle diese Ansätze haben aber bisher nicht zu einem entscheidenden Durchbruch in Hinblick auf ein konkurrenzfähiges Marktprodukt geführt.

Es besteht daher nach wie vor die Aufgabe, Oxidationsfärbemittel zu entwickeln, die aufgrund ihres geringeren Gehaltes an Oxidationsmitteln oder durch Oxidation mit Luftsauerstoff schonendere Färbungeverfahren ermöglichen, ohne das Färbeergebnis negativ zu beeinflussen. Weiterhin ist es in hohem Maße wünschenswert, den pH-Wert der Färbemittel auf einen Wert um den Neutralpunkt zu senken, um möglichen Schädigungen der Faser aufgrund der üblicherweise starken Alkalität der Färbemittel vorzubeugen.

Der Einsatz von Übergangsmetallkomplexen im Zusammenhang mit einer oxidativen Haarfärbung ist prinzipiell bekannt. So wurde in der europäischen Patentanmeldung EP-A2-507 448 vorgeschlagen, die Haare vor dem eigentlichen Färbeprozeß mit einem Übergangsmetallkomplex mit den Liganden 2,2'-Dipyridyl oder o-Phenanthrolin zu behandeln, um das Haar gegen einen oxidativen Abbau des Cysteins zu stabilisieren. In der deutschen Patentschrift DE-C1-195 34 214 wurde weiterhin vorgeschlagen, Übergangsmetallkomplexe als Oxidationskatalysatoren in Haarfärbemitteln einzusetzen, die als Entwicklerkomponenten Derivate des 4-(2,5-Diaminophenoxymethyl)-1,3-dioxolans enthalten. Schließlich wurde in der deutschen Anmeldung DE-197 57 510.2 vorgeschlagen, Übergangsmetallkomplexe mit Liganden vom Salen-Typ als Oxidationskatalysatoren einzusetzen. In allen diesen Färbepräperaten sind die Übergangsmetallkationen in freier Form enthalten und können sich potentiell am oder im Haar anlagern. Dies ist jedoch hinsichtlich einer Minimierung des Schädigungspotentials der Färbemittel nicht als optimal anzusehen. Daher bestand die Aufgabe Oxidationskatalysatoren für Haarfärbemittel zu cntwickeln, die nur zu einer sehr geringen Konzentration freier Übergangsmetallkationen in der Anwendungszubereitung führen.

Es wurde nunmehr überraschenderweise gefunden, daß bei Verwendung von in Hohlräumen von Käfigverbindungen eingeschlossenen Übergangsmetallkomplexen in Oxidationsfärbemitteln
- die Menge des Oxidationsmittels stark reduziert werden kann und/oder
- die Ausfärbung alleine mit Luftsauerstoff als Oxidationsmittel erfolgen kann oder
- die Ausfärbung im neutralen pH-Bereich erfolgen kann.
Die erfindungsgemäßen Mittel zeichnen sich weiterhin durch die geringe Konzentration freier Übergangsmetallkationen in der Anwendungszubereitung aus.

Ein erster Gegenstand der Erfindung sind somit Mittel zum Färben keratinischer Fasern, insbesondere menschlicher Haare, die mindestens ein Farbstoffvorprodukt enthalten sowie 0,0001 bis 1,0 Gew.-% eines in Hohlräumen von Käfigverbindungen eingeschlossenen Übergangsmetallkations mit mindestens zwei stabilen Oxidationsstufen, das mit mindestens einem mehrzähnigen Liganden komplexiert ist.

Mehrzähnige Liganden sind erfindungsgemäß Moleküle, die zwei oder mehrere Elektronendonatorzentren aufweisen.

Bevorzugte Übergangsmetalle sind Eisen, Kobalt, Kupfer, Mangan, Molybdän, Ruthenium und/oder Vanadium; Komplexe mit Eisen und insbesondere mit Kupfer oder Ruthenium als Übergangsmetall haben sich als erfindungsgemäß besonders geeignet erwiesen.

Zu den bevorzugten Käfigverbindungen, in welche die Übergangsmetallkomplexe eingeschlossen werden, gehören die Zeolithe vom Typ A, K, L, P-L, O, T, X, Y und Ω sowie deren Mischungen, wobei die Auswahl der geeigneten Käfigverbindung sich nach der Größe des einzuschließenden Übergangsmetallkomplexes richtet. Dabei ist maßgeblich, daß im Rahmen der Herstellung der eingeschlossenen Komplexe sowohl das Übergangsmetallion wie auch die für die Komplexbildung notwendigen Liganden in den Hohlraum der Käfigverbindung gelangen können, der sich bildende Komplex aber nicht mehr durch die Öffnung des Hohlraums passen darf, damit ein stabiler Einschluß des Komplexes gewährleistet ist. Die Größen der Hohlräume der verschiedenen Zeolithe sind dem Fachmann prinzipiell bekannt. So besitzt beispielsweise der erfindungsgemäß bevorzugte Zeolith Y Hohlräume mit Durchmessern von ca. 1,3 nm, deren Öffnungen einen Durchmesser von 0,74 nm aufweisen. Unter einem stabilen Einschluß soll dabei verstanden werden, daß unter Anwendungsbedingungen, das heißt im Haarbehandlungsmitttel, keine die Oxidationskatalyse beeinflussenden Mengen an freiem Komplex nachweisbar sind. Die sich innerhalb der Hohlräume von Käfigverbindungen bildenden und dann eingeschlossenen Übergangsmetallkomplexe sind wesentlich stabiler und damit über einen längeren Zeitraum wirksam als die gleichen Übergangsmetallkomplexe, die auf die Oberfläche von Trägermaterialien aufgebracht worden sind, auch wenn es sich bei diesen Trägermaterialien um die gleichen Käfigverbindungen handelt.

Als Liganden in den erfindungsgemäß brauchbaren Übergangsmetallkomplexen kommen übliche Substanzen sowohl anorganischer als auch organischer Natur in Frage. Zu den organischen Liganden in derartigen Komplexen gehören neben Carboxylaten insbesondere Verbindungen mit primären, sekundären und/oder tertiären Amin- und/oder Alkohol-Funktionen, wie Pyridin, Pyridazin, Pyrimidin, Pyrazin, Imidazol, Pyrazol, Triazol, 2,2'-Bispyridylamin, Tris-(2-pyridylmethyl)amin, 1,4,7-Triazacyclononan, 1,4,7-Trimethyl-1,4,7-triazacyclononan, 1,5,9-Trimethyl-1,5,9-triazacyclododecan, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimidazolylmethyl)aminomethyl)-4-methylphenol, N,N,N',N'-Tetrakis-(2-benzimidazolylmethyl)-2-hydroxy-1,3-diaminopropan, 2,6-Bis-(bis-(2-pyridylmethyl)aminomethyl)-4-methylphenol, 1,3-Bis-(bis-(2-benzimidazolylmethyl)aminomethyl)-benzol, Sorbitol, Mannitol, Erythritol, Adonitol, Inositol, Lactose, und gegebenenfalls substituierte Salene, Porphine und Porphyrine. Zu den anorganischen Neutralliganden gehören insbesondere Ammoniak und Wasser. Insbesondere bei den Co(III)-Komplexen, in denen das Zentralatom normalerweise mit der Koordinationszahl 6 vorliegt, ist die Anwesenheit von mindestens 1 Ammoniak-Liganden bevorzugt. Falls nicht sämtliche Koordinationsstellen des Übergangsmetallzentralatoms durch Neutralliganden besetzt sind, enthält ein gemäß der Erfindung zu verwendender Komplex weitere, vorzugsweise anionische und unter diesen insbesondere ein- oder zweizähnige Liganden. Zu diesen gehören insbesondere die Halogenide wie Fluorid, Chlorid, Bromid und Iodid, und die (NO₂)⁻-Gruppe. Unter einer (NO₂)⁻-Gruppe soll im vorliegenden Fall ein Nitro-Ligand, der über das Stickstoffatom an das Übergangsmetall gebunden ist, oder ein Nitrito-Ligand, der über ein Sauerstoffatom an das Übergangsmetall gebunden ist, verstanden werden. Die (NO₂)⁻-Gruppe kann an ein Übergangsmetall auch chelatbildend gebunden sein oder sie kann zwei Übergangsmetallatome asymmetrisch oder µ¹-O-verbrücken. Außer den genannten Liganden können die im Aktivatorsystem gemäß der Erfindung zu verwendenden Übergangsmetallkomplexe noch weitere, in der Regel einfacher aufgebaute Liganden, insbesondere ein- oder mehrwertige Anionliganden, tragen. In Frage kommen beispielsweise Nitrat, Actetat, Trifluoracetat, Formiat, Carbonat, Citrat, Perchlorat sowie komplexe Anionen wie Hexafluorophosphat. Die Anionliganden sollen für den Ladungsausgleich zwischen Übergangsmetall-Zentralatom und dem Ligandensystem sorgen. Auch die Anwesenheit von Oxo-Liganden, Peroxo-Liganden und Imino-Liganden ist möglich. Insbesondere derartige Liganden können auch verbrückend wirken, so daß mehrkernige Komplexe entstehen. Im Falle verbrückter, zweikerniger Komplexe müssen nicht beide Metallatome im Komplex gleich sein. Auch der Einsatz zweikerniger Komplexe, in denen die beiden Übergangsmetallzentralatome unterschiedliche Oxidationszahlen aufweisen, ist möglich.

Falls Anionliganden fehlen oder die Anwesenheit von Anionliganden nicht zum Ladungsausgleich im Komplex führt, können in den gemäß der Erfindung zu verwendenden Übergangsmetallkomplex-Verbindungen anionische Gegenionen anwesend sein, die den kationischen Übergangsmetall-Komplex neutralisieren. Zu diesen anionischen Gegenionen gehören insbesondere Nitrat, Hydroxid, Hexafluorophosphat, Sulfat, Chlorat, Perchlorat, die Halogenide wie Chlorid oder die Anionen von Carbonsäuren wie Formiat, Acetat, Benzoat oder Citrat.

Erfindungsgemäß bevorzugt sind Liganden, die mindestens ein Stickstoffatom als Koordinationsstelle aufweisen.

In einer Ausführungsform der vorliegenden Erfindung können Liganden bevorzugt sein, die mindestens eine nicht-aromatische Koordinationsstelle aufweisen. Diese Liganden können besser in die Hohlräume der Käfigverbindungen eindringen und somit ist die Bildung der erfindungsgemäßen Katalysatoren begünstigt. Besonders bevorzugte Liganden sind gemäß dieser Ausführungsform:
- 1,4,8,11-Tetraazacyclotetradecan
- 1,8-Diazabicyclo[5.4.0]undec-7-en (1,5-5)
- Ethylendiamin
- Nitrilotriessigsäure
- Tris(aminoethyl)amin
- Tris(aminomethyl)methan
- 1,3,5-Triaminocyclohexan sowie
- Pyridin-2,6-dicarbonsäure.

Gemäß einer zweiten bevorzugten Ausführungsform der vorliegenden Erfindung können Liganden bevorzugt sein, die mindestens eine aromatische Koordinationsstelle aufweisen. Die erfindungsgemäßen Übergangsmetallkomplexe mit aromatischen Liganden zeichnen sich durch eine erhöhte Stabilität aus und ermöglichen somit eine breitere Anwendbarkeit. Besonders bevorzugte Liganden mit mindestens einer aromatischen Koordinationsstelle sind beispielsweise
- 2,2':6',2"-Terpyridin
- 1,10-Phenanthrolin
- Tris(2-pyridylmethyl)amin
- Tris(2-pyridylethyl)amin sowie
- N-Carboxymethyl-N-(2-pyridylmethyl)glycin.

In einer ersten Ausführungsform der vorliegenden Erfindung kann das Farbstoffvorprodukt ein Oxidationsfarbstoffvorprodukt vom Typ Entwickler sein. Es können auch mehrere Entwickler gemeinsam in den erfindungsgemäßen Mitteln eingesetzt werden.

Entwicklersubstanzen sind üblicherweise aromatische oder heterocyclische Ringsysteme, die durch zwei in ortho- oder para-Stellung zueinander stehende reaktive Gruppen, i.a. Hydroxy- oder Aminogruppen, gekennzeichnet sind. Solche Verbindungen sind beispielsweise primäre aromatische Amine mit einer weiteren, in para- oder ortho-Position befindlichen freien oder substituierten Hydroxy- oder Aminogruppe, ferner Diaminopyridinderivate, heterocyclische Hydrazonderivate oder 4-Aminopyrazolonderivate.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Aminomethyl-4-aminophenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.

Eine weitere bevorzugt Entwicklerkomponente ist 4-Amino-2-((diethylamino)-methyl)-phenol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraammopyrimidin, 2-Hydroxy-4,5,6-triaminopyrimidin sowie 4-Hydroxy-2,5,6-triaminopyrimidin.

Zur Nuancierung der erzielbaren Farbtöne können die erfindungsgemäßen Mittel weiterhin noch eine oder mehrere Kupplerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind 1-Naphthol, Pyrogallol, 1,5-, 2,7-und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kuppler-Komponenten sind 1-Naphthol, 1,5-, 2,7- und 1,7-Dihydroxynaphthalin, 5-Amino-2-methylphenol, Resorcin, 3-Aminophenol, 4-Chlorresorcin, 2-Chlor-6-methyl-3-aminophenol, 2-Methylresorcin, 5-Methylresorcin, 2,5-Dimethylresorcin sowie 2,6-Dihydroxy-3,4-diaminopyridin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer zweiten bevorzugten Ausführungsform des Gegenstandes der vorliegenden Erfindung kann das Farbstoffvorprodukt ein Derivat des Indolins der Formel (Ia) sein, 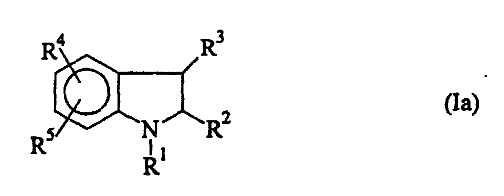 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, daß R⁴ und R⁵ nicht gleichzeitig Wasserstoff sind.

In einer dritten bevorzugten Ausführungsform dieses Gegenstandes der vorliegenden Erfindung kann das Farbstoffvorprodukt ein Derivat des Indols der Formel (Ib) sein, 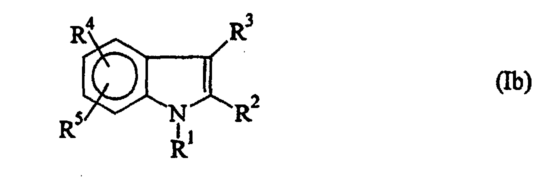 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, daß R⁴ und R⁵ nicht gleichzeitig Wasserstoff sind.

Bevorzugte Stoffe der Formel (Ia) sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Bevorzugte Stoffe der Formel (Ib) sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Ganz besonders bevorzugt sind 5,6-Dihydroxyindol sowie 5,6-Dihydroxyindolin.

In einer ersten bevorzugten Variante der oben beschriebenen Ausführungsformen werden die Mittel derart formuliert, daß sie als Farbstoffvorprodukte nur Indol- und/oder Indolinderivate der Formeln (Ia) und (Ib) enthalten und frei sind von üblichen Oxidationsfarbstoffvorprodukten vom Entwickler- bzw. Kupplertyp.

In einer zweiten bevorzugten Variante der oben beschriebenen Ausführungsformen können die erfindungsgemäßen Mittel neben den Indol- und/oder Indolinderivaten der Formeln (Ia) und (Ib) auch noch übliche Oxidationsfarbstoffvorprodukte vom Entwickler- bzw. Kupplertyp enthalten.

Es kann erfindungsgemäß besonders bevorzugt sein, die Indol- und/oder die Indolinderivate der Formeln (Ia) und (Ib) in Kombination mit einer oder mehrere Kupplerkomponenten in Haarfärbemitteln einzusetzen. Beispielhaft sei an dieser Stelle ausdrücklich auf die oben genannten Kupplerkomponenten verwiesen.

Weiterhin kann es erfindungsgemäß bevorzugt sein, die Indol- und/oder Indolinderivate der Formel (la) und (Ib) in Kombination mit mindestens einer Aminosäure oder einem Oligopeptid in Haarfärbemitteln einzusetzen. Es kann erfndungsgemäß weiterhin bevorzugt sein, wenn die Aminosäure eine α-Aminosäure ist. Ganz besonders bevorzugte α-Aminosäuren sind Arginin, Ornithin, Lysin und Histidin.

In einer bevorzugten Ausführungsform enthalten die erfindungsgemäßen Haarfärbemittel zur weiteren Modifizierung der Farbnuancen neben den Farbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Farbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbemitteln einseizbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Färbungen von besonderer Farbtiefe können erreicht werden, wenn die Mittel neben den Farbstoffen und/oder Farbstoffvorprodukten zusätzlich noch ein Öl des Wiesenschaumkrauts (INCI-Bezeichnung: Meadowfoam Seed Oil) enthalten.

Zur Herstellung der erfindungsgemäßen Färbemittel werden die Farbstoffvorprodukte in einen geeigneten wäßrigen, alkoholischen oder wäßrig-alkoholischen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen.

Die erfindungsgemäßen Färbemittel können weiterhin alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Färbemitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkyloplyethylen-und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈- bis C₂₂-Carbonsäuren, wie Ölsäure, Stearinsäure, Isostearinsäure und Palmitinsäure. Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂- bis C₂₂-Fettsäuremono- und -diester von Anlagerungsprodukten von 1 bis 30 Mol Ethylenoxid an Glycerin,
- C₈- bis C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, bei-spielsweise das Kokosalkyl-dimethylammoniumglycinat, N-Acyl-aminopropyl-N,N-dimethyl-ammoniumglycinate, beispielsweise das Kokosacylaminopropyl-dimethyl-anmionium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈- bis C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldmiethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Methylhydroxyalkyl-dialkoyloxyalkyl-ammonium-methosulfate sowie die unter dem Warenzeichen Dehyquart® vertriebenen Produkte.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Je nach Art des Mittels und des Tensidtyps sind die Tenside in den erfindungsgemäßen Mitteln üblicherweise in Mengen von insgesamt 0,5 bis 30 Gew.-%, bezogen auf das gesamte Mittel, enthalten.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten. Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen.

Als konditionierende Wirkstoffe weiterhin geeignet sind Silikonöle, insbesondere Dialkylund Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Schließlich enthalten die erfindungsgemäßen Färbemittel bevorzugt noch einen Fettstoff.

Bevorzugte Fettstoffe sind lineare und verzweigte, gesättigte und ungesättigte Fettalkohole oder natürliche Fettalkoholgemische mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Ebenfalls als Fettstoffe eingesetzt werden können Monoester der Fettsäuren mit Alkoholen mit 6 bis 24 C-Atomen sowie Triglyceride natürlichen Ursprungs.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylanud/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- symmetrische und unsymmetrische, lineare und verzweigte Dialkylether mit insgesamt zwischen 12 bis 36 C-Atomen, insbesondere 12 bis 24 C-Atomen, beispielsweise Din-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- Antischuppenwirkstoffe wie Piroctone Olamine, Zink Omadine und Climbazol,
- tierische und pflanzliche Proteinhydrolysate, insbesondere Elastin-, Kollagen-, Keratin-, Milcheiweiß-, Sojaprotein-, Mandelprotein- und Weizenproteinhydrolysate, sowie deren Fettsäurekondensationsprodukte und quaternierte Derivate,
- Vitamine und Vitaminvorstufen wie Panthenol, dessen Derivate und Biotin,
- Pflanzen- und Honigextrakte, wie insbesondere Extrakte aus Eichenrinden, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel,
- Weitere Wirkstoffe wie Ceramide, Allantoin, Pyrrolidoncarbonsäuren, und Bisabolol,
- Lichtschutzmittel,
- Entschäumer wie Silikone,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Cholesterin,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Konsistenzgeber wie Zuckerester, Polyolester oder Polyolalkylether,
- Strukturanten wie Maleinsäure, Mono-, Di- und Oligosaccharide,
- Fette und Wachse, wie Walrat, Bienenwachs, Montanwachs und Paraffine,
- Fettsäurealkanolamide,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, N₂O, Dimethylether, CO₂, N₂ und Luft Bezüglich weiterer Bestandteile sowie Mengenbereiche für die einzelnen Inhaltsstoffe wird auf die dem Fachmann bekannten Handbücher, z.B. K. Schrader, Grundlagen und Rezepturen der Kosmetika, 2. Auflage, Hüthig Buch Verlag, Heidelberg, 1989, verwiesen.

Die oxidative Entwicklung der Färbung kann grundsätzlich allein mit Luftsauerstoff erfolgen. Es soll jedoch nicht ausgeschlossen weden, daß auch ein chemisches Oxidationsmittel eingesetzt wird, besonders dann, wenn neben der Färbung ein Aufhelleffekt am menschlichen Haar gewünscht ist. Als Oxidationsmittel kommen insbesondere Wasserstoffperoxid oder dessen Anlagerungsprodukte an Harnstoff, Melamin oder Natriumborat in Frage. Weiterhin ist es möglich, die Oxidation mit Hilfe von Enzymen durchzuführen. Dabei können die Enzyme sowohl zur Erzeugung von oxidierenden Perverbindungen eingesetzt werden, als auch zu Verstärkung der Wirkung einer geringen Menge vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Die erfindungsgemäßen Oxidationskatalysatoren können erfindungsgemäß sowohl in einer gemeinsamen Zubereitung mit den Farbstoffvorprodukten als auch separat konfektioniert sein.

In einer bevorzugten Ausführungsform der erfindungsgemäßen Lehre werden die Oxidationskatalysatoren separat in einem geeigneten Lösemittel, beispielsweise in Wasser, Ethanol oder Aceton, gelöst beziehungsweise suspendiert und unmittelbar vor dem Färben der Haare mit der Oxidationsmittelzubereitung verrührt. Diese Zubereitung wird anschließend mit einer Zubereitung, enthaltend die Farbstoffvorprodukte, vermischt. Das dabei entstehende gebrauchsfertige Haarfärbepräparat sollte bevorzugt einen pH-Wert im Bereich von 6 bis 10 aufweisen. Besonders bevorzugt ist ein pH-Wert von 6,5 bis 8. Die Anwendungstemperaturen können in einem Bereich zwischen 15 und 40°C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert.

Ein zweiter Gegenstand der vorliegenden Erfindung ist die Verwendung der obengenannten Mittel zur Färbung keratinischer Fasern.

Durch die erfindungsgemäße Verwendung der Übergangsmetall-Komplexe ist es möglich
- die üblicherweise nur unter alkalischen Bedingungen erhaltenen Färbeleistungen auch im neutralen pH-Bereich zu erzielen oder
- eine schonendere Färbebehandlung dadurch zu erzielen, daß im alkalischen Bereich die angestrebte Färbeleistung bereits ohne Oxidationsmittel beziehungsweise mit deutlich, d.h. bis zu 75 %, niedrigeren Konzentrationen erreicht wird.

### Beispiele

### 1. Herstellung der Katalysatoren

100g Zeolith Y wurden mit ca. 1000ml einer 1 Gew.-%igen Kupfer(II)chloridlösung 3 bis 5 Stunden bei Raumtemperatur gerührt. Anschließend wurde der Zeolith abgesaugt und mit Wasser chloridfrei gewaschen (Nachweis mit salpetersaurer AgNO₃-Lösung). Um den Zeolith von äußerlich anhaftendem Metallionen zu befreien, wurde der metalldotierte Zeolith mehrfach mit einer 5 Gew.-%igen Natriumchloridlösung gewaschen. Der Rückaustausch ist beendet, wenn im Filtrat kein Metall mehr nachweisbar ist. Anschließend wurde der Zeoltih erneut chloridfrei gewaschen, 18 Stunden bei 110°C in einem Umlufttrockenschrank getrocknet und schließlich bei 250°C bis zur Gewichtskonstanz calciniert.

Der Kupfergehalt des derart metalldotierten Zeoliths wurde mittels Röntgenfluoreszenzanalyse bestimmt. Anschließend wurde der metalldotierte Zeolith mit 1,8-Diazablcyclo[5.4.0]undec-7-en (1,5-5) (kurz: DBU) versetzt, so daß sich ein molares Verhältnis Cu: DBU von 1:2,5 ergab, und bei 80°C mehrere Stunden im Wasserstrahlvakuum (80-100mbar) am Rotavapor gerührt. Anschließend wurde der Überschuß DBU mit Wasser bis zum Neutralpunkt ausgewaschen und das Produkt 8 Stunden bei 110°C in einem Umlufttrockenschrank getrocknet.

Das Produkt wies folgende Zusammensetzung auf:
- 4,14 Gew.-% Kohlenstoff(C)
- 1,03 Gew.-% Stickstoff (N)
- 1,10 Gew.-% Kupfer (Cu)

Dies entspricht einem Molverhältnis C : N : Cu von 19,9 : 4,3 : 1 und damit einem molaren Verhältnis von Cu : DBU von 1:2.

### 2. Herstellung der Färbecreme

Es wurde eine Färbecreme folgender Zusammensetzung hergestellt (alle Angaben sind, soweit nicht anders vermerkt, in g):

| **Teilmischung A** | |
|---|---|
| Hydrenol® D¹ | 8,50 |
| Lorol® techn.² | 2,00 |
| Eumulgin® B2³ | 0,75 |
| Texapon® NSO⁴ | 20,00 |
| Dehyton® K⁵ | 12,50 |
| Wasser | 30,00 |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Cetylstearylalkohol mit ca. 20 EO-Einheiten (INCI-Bezeichnung: Ceteareth-20) (HENKEL) | |
| ⁴ Laurylethersulfat, Natriumsalz (ca. 27,5% Aktivsubstanz; INCI-Bezeichnung: Sodium Laureth Sulfate) (HENKEL) | |
| ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (HENKEL) | |

Die Substanzen Hydrenol D, Lorol und Eumulgin B2 wurden bei 80°C aufgeschmolzen, mit dem 80°C heißen Wasser, enthaltend Texapon NSO und Dehyton K, vermischt und unter starkem Rühren emulgiert. Danach wurde die Emulsion unter schwachem Rühren abgekühlt.

| **Teilmischung B** | |
|---|---|
| Natriumsulfit | 1,00 |
| Ammoniumsulfat | 1,00 |
| 5-Amino-2-methylphenol | 0,003mol |
| 4-Amino-2-aminomethylphenol-hydrochlorid | 0,003mol |
| Ammoniak (25%ige Lösung) | ad pH |
| Wasser | 10,00 |

Die Farbstoffvorprodukte wurden in dem 50°C heißem Wasser unter Zugabe von Natriumsulfit, Ammoniumsulfat und Ammoniak gelöst. Die Farbstoffvorproduktlösung (Teilmischung B) wurde zur Emulsion (Teilmischung A) gegeben, mit einer Ammoniaklösung auf den gewünschten pH-Wert eingestellt und mit Wasser auf 100 Gewichtsteile aufgefüllt. Es wurde bis zum Erreichen der Raumtemperatur weitergerührt.

### 3. Ausfärbungen

Als Entwickler wurden gegebenenfalls 10 ml einer 3 Gew.-%igen polyacrylathaltigen, wäßrigen Lösung von Wasserstoffperoxid verwendet. Der erfindungsgemäße Katalysator wurde in einigen Tropfen Wasser suspendiert, gegebenenfalls zu der Entwicklerlösung gegeben und anschließend in die Färbecreme eingearbeitet

Anschließend wurde die Anwendungszubereitung auf eine Haarsträhne der Sorte "Kerling Naturweiß" (1,5 g Anwendungszubereitung pro g Haar), gegeben, dort 30 Minuten belassen und dann mit Wasser ausgespült.

Es wurden die folgenden Ausfärbungen unter den jeweils angegebenen Bedingungen durchgeführt:

| Metall | Konzentration | Ligand | H₂O₂-Konzentration | pH-Wert | Bewertung |
|---|---|---|---|---|---|
| Ruthenium | 200 ppm | DBU | 0,35 Gew.-% | 9,0 | Vollausfärbung |
| Ruthenium | 200 ppm | DBU | 1,5 Gew.-% | 7,0 | Vollausfärbung |
| Kupfer | 200 ppm | DBU | --- | 9,0 | Vollausfärbung |
| Kupfer | 200 ppm | DBU | 1,5 Gew.-% | 7,0 | Vollausfärbung |
| --- | --- | --- | 1,5 Gew.-% | 7,0 | Keine Färbung |
| --- | --- | --- | 1,5 Gew.-% | 9,0 | Vollausfärbung |

## Patentansprüche

1. Mittel zum Färben keratinischer Fasern, insbesondere menschlicher Haare, enthaltend mindestens ein Farbstoffvorprodukt, **dadurch gekennzeichnet, daß** es 0,0001 bis 1,0 Gew.-% eines in Hohlräumen von Käfigverbindungen eingeschlossenen Übergangsmetallkations mit mindestens zwei stabilen Oxidationsstufen, das mit mindestens einem mehrzähnigen Liganden komplexiert ist, enthält.

2. Mittel nach Anspruch 1, **dadurch gekennzeichnet, daß** das Übergangsmetallkation ausgewählt ist aus einer Gruppe gebildet von Eisen, Kobalt, Kupfer, Mangan, Molybdän, Ruthenium und Vanadium.

3. Mittel nach Anspruch 2, **dadurch gekennzeichnet, daß** das Übergangsmetallkation Kupfer oder Ruthenium ist.

4. Mittel nach einem der Ansprüche 1 bis 3, **dadurch gekennzeichnet, daß** die Käfigverbindungen, in welche die Übergangsmetallkationen eingeschlossen werden, aus den Zeolithen vom Typ A, K, L, P-L, O, T, X, Y und Ω sowie deren Mischungen ausgewählt werden.

5. Mittel nach einem der Ansprüche 1 bis 4, **dadurch gekennzeichnet, daß** der Ligand mindestens ein Stickstoffatom als Koordinationsstelle aufweist.

6. Mittel nach einem der Ansprüche 1 bis 5, **dadurch gekennzeichnet, daß** der Ligand ausgewählt ist aus einer Gruppe gebildet von
- 1,4,8,11-Tetraazacyclotetradecan
- 1,8-Diazabicyclo[5.4.0]undec-7-en (1,5-5)
- Ethylendiamin
- Nitrilotriessigsäure
- Tris(aminoethyl)amin
- Tris(aminomethyl)methan
- 1,3,5-Triaminocyclohexan sowie
- Pyridin-2,6-dicarbonsäure.

7. Mittel nach einem der Ansprüche 1 bis 6, **dadurch gekennzeichnet, daß** der Ligand mindestens eine aromatische Koordinationsstelle aufweist.

8. Mittel nach Anspruch 7, **dadurch gekennzeichnet, daß** der Ligand mit mindestens einer aromatischen Koordinationsstelle aus einer Gruppe stammt, die gebildet wird von
- 2,2':6',2"-Terpyridin
- 1,10-Phenanthrolin
- Tris(2-pyridylmethyl)amin
- Tris(2-pyridylethyl)amin sowie
- N-Carboxymethyl-N-(2-pyridylmethyl)glycin.

9. Mittel nach einem der Ansprüche 1 bis 8, **dadurch gekennzeichnet, daß** es mindestens ein Farbstoffvorprodukt vom Typ der Entwickler enthält.

10. Mittel nach einem der Ansprüche 1 bis 9, **dadurch gekennzeichnet**, es als Farbstoffvorprodukt mindestens eine Indol- und/oder Indolinderivat enthält.

11. Mittel nach einem der Ansprüche 1 bis 10, **dadurch gekennzeichnet, daß** es mindestens eine Kupplerkomponente enthält.

12. Mittel nach einem der Ansprüche 1 bis 11, **dadurch gekennzeichnet, daß** es einen pH-Wert von 6,5 bis 8 aufweist.

13. Mittel nach einem der Ansprüche 1 bis 12, **dadurch gekennzeichnet, daß** es keine chemischen Oxidationsmittel enthält.

14. Verwendung eines der Mittel der Ansprüche 1 bis 13 zum Färben keratinischer Fasern.

## Claims

1. A composition containing at least one dye precursor for coloring keratin fibers, more particularly human hair, **characterized in that** it contains 0.0001 to 1.0% by weight of a transition metal cation with at least two stable oxidation stages included in cavities of clathrate compounds which is complexed with at least one multidentate ligand.

2. A composition as claimed in claim 1, **characterized in that** the transition metal cation is selected from the group consisting of iron, cobalt, copper, manganese, molybdenum, ruthenium and vanadium.

3. A composition as claimed in claim 2, **characterized in that** the transition metal cation is copper or ruthenium.

4. A composition as claimed in any of claims 1 to 3, **characterized in that** the clathrate compounds in which the transition metal cations are included are selected from zeolites of type A, K, L, P-L, O, T, X, Y and Ω and mixtures thereof.

5. A composition as claimed in any of claims 1 to 4, **characterized in that** the ligand has at least one nitrogen atom as a coordination site.

6. A composition as claimed in any of claims 1 to 5, **characterized in that** the ligand is selected from the group consisting of
- 1,4,8,11-tetraazacyclotetradecane
- 1,8-diazabicyclo[5.4.0]undec-7-ene (1,5-5)
- ethylenediamine
- nitrilotriacetic acid
- tris(aminoethyl)amine
- tris(aminomethyl)methane
- 1,3,5-triaminocyclohexane and
- pyridine-2,6-dicarboxylic acid.

7. A composition as claimed in any of claims 1 to 6, **characterized in that** the ligand has at least one aromatic coordination site.

8. A composition as claimed in claim 7, **characterized in that** the ligand with at least one aromatic coordination site comes from a group consisting of
- 2,2':6',2"-terpyridine
- 1,10-phenanthroline
- tris(2-pyridylmethyl)amine
- tris(2-pyridylethyl)amine and
- N-carboxymethyl-N-(2-pyridylmethyl)glycine.

9. A composition as claimed in any of claims 1 to 8, **characterized in that** it contains at least one dye precursor of the primary intermediate type.

10. A composition as claimed in any of claims 1 to 9, **characterized in that** it contains at least one indole and/or indoline derivative as dye precursor.

11. A composition as claimed in any of claims 1 to 10, **characterized in that** it contains at least one secondary intermediate.

12. A composition as claimed in any of claims 1 to 11, **characterized in that** it has a pH value of 6.5 to 8.

13. A composition as claimed in any of claims 1 to 12, **characterized in that** it does not contain any chemical oxidizing agents.

14. The use of one of the compositions claimed in claims 1 to 13 for coloring keratin fibers.

## Revendications

1. Agent destiné à la teinture de fibres kératiniques, en particulier de cheveux humains, contenant au moins un précurseur de colorant, **caractérisé en ce qu'**il contient de 0,0001 à 1,0 % en poids d'un cation de métal de transition ayant au moins deux degrés d'oxydation stables, inclus dans les interstices de composés complexants, qui est complexé avec au moins un ligand multidenté.

2. Agent selon la revendication 1, **caractérisé en ce que** le cation de métal de transition est choisi dans le groupe constitué par le fer, le cobalt, le cuivre, le manganèse, le molybdène, le ruthénium et le vanadium.

3. Agent selon la revendication 2, **caractérisé en ce que** le cation de métal de transition est le cuivre ou le ruthénium.

4. Agent selon l'une quelconque des revendications 1 à 3, **caractérisé en ce que** les composés complexants, dans lesquels les cations de métal de transition sont inclus, sont choisis parmi les zéolithes de type A, K, L, P-L, O, T, X, Y et Ω, ainsi que leurs mélanges.

5. Agent selon l'une quelconque des revendications 1 à 4, **caractérisé en ce que** le ligand présente au moins un atome d'azote comme site de coordination.

6. Agent selon l'une quelconque des revendications 1 à 5, **caractérisé en ce que** le ligand est choisi dans le groupe constitué par les composés suivants :
- 1,4,8,11-tétraazacyclotétradécane
- 1,8-diazablcyclo[5.4.0]undéc-7-ène (1,5-5)
- éthylènediamine
- acide nitrilotriacétique
- tris(aminoéthyl)amine
- tris(aminométhyl)méthane
- 1,3,5-triaminocyclohexane
- acide pyrlndine-2,6-dicarboxylique.

7. Agent selon l'une quelconque des revendications 1 à 6, **caractérisé en ce que** le ligand présente au moins un site de coordination aromatique.

8. Agent selon la revendication 7, **caractérisé en ce que** le ligand qui présente au moins un site de coordination aromatique appartient au groupe constitué par les composés suivants :
- 2,2':6',2"-terpyridine
- 1,10-phénanthroline
- tris(2-pyridylméthyl)amine
- tris(2-pyridyléthyl)amine et
- N-carboxyméthyl-N-(2-pyridylméthyl)glycine.

9. Agent selon l'une quelconque des revendications 1 à 8, **caractérisé en ce qu'**il contient au moins un précurseur de colorant de type développeur.

10. Agent selon l'une quelconque des revendications 1 à 9, **caractérisé en ce que** comme précurseur de colorant, il contient un dérivé d'indol et/ou d'indoline.

11. Agent selon l'une quelconque des revendications 1 à 10, **caractérisé en ce qu'**il contient au moins un composant de couplage.

12. Agent selon l'une quelconque des revendications 1 à 11, **caractérisé en ce qu'**il présente un pH de 6,5 à 8.

13. Agent selon l'une quelconque dès revendications 1 à 12, **caractérisé en ce qu'**il ne contient pas d'agent oxydant chimique.

14. Utilisation d'un agent selon les revendications 1 à 13 pour la teinture de fibres kératiniques.
